# EUROPEAN PATENT SPECIFICATION

(11) **EP 3 331 991 B1**
(45) Date of publication and mention of the grant of the patent: **20.07.2022**
(21) Application number: 16766606.4
(22) Date of filing: 28.07.2016
(51) Int. Cl.: C12N 5/077, A01N 1/02

(54) **CELLULAR CULTURE MEDIUM FREE FROM SERUM.**
SERUMFREIES ZELLKULTURMEDIUM
MILIEU DE CULTURE CELLULAIRE EXEMPT DE SÉRUM

(30) Priority: 03.08.2015 IT UB20152804
(43) Date of publication of application: 13.06.2018
(73) Proprietor: Blast Research S.r.l., 20121 Milano (IT)
(72) Inventor: COSTA, Massimo, 20121 Milano (IT); PASSI, Alberto Giuseppe, 20121 Milano (IT)
(74) Representative: Perani & Partners S.p.A.
(86) International application number: PCT/IB2016/054519
(87) International publication number: WO 2017/021831

(56) References cited:
- Alexis Carrel ET AL: "ACTION ON FIBROBLASTS OF EXTRACTS OF HOMOL- OGOUS AND HETEROLOGOUS TISSUES", Anat. Rec. Anat. Rec. J. Exp. Med.. Med. J. Exp. Med. Exp. Med. Exp. Med. Y. Exp. Med.Exp. Med.Exp. Med, 1 January 1921 (1921-01-01), pages 759-1916, XP055263349, Retrieved from the Internet: URL:http://jem.rupress.org/content/38/5/49 9.full.pdf#page=1&view=FitH
- STACY R SMITH ET AL: "A multicenter, double-blind, placebo-controlled trial of autologous fibroblast therapy for the treatment of nasolabial fold wrinkles", DERMATOLOGIC SURGERY, WILEY-BLACKWELL PUBLISHING, INC, NEW YORK, US , vol. 38, no. 7 1 July 2012 (2012-07-01), pages 1234-1243, XP002714258, ISSN: 1076-0512, DOI: 10.1111/J.1524-4725.2012.02349.X Retrieved from the Internet: URL:http://onlinelibrary.wiley.com/doi/10. 1111/j.1524-4725.2012.02349.x/pdf [retrieved on 2012-03-12]
- VADIM ZORIN ET AL: "Clinical-instrumental and morphological evaluation of the effect of autologous dermal fibroblasts administration", JOURNAL OF TISSUE ENGINEERING AND REGENERATIVE MEDICINE, 19 December 2014 (2014-12-19), pages n/a-n/a, XP055263248, US ISSN: 1932-6254, DOI: 10.1002/term.1976 cited in the application
- "Formulation for Iscove's Modified Dulbecco's Medium (IMDM) ATCC", , 8 April 2016 (2016-04-08), XP055263745, Retrieved from the Internet: URL:http://www.lgcstandards-atcc.org/~/med ia/Attachments/E/9/7/E/4893.ashx [retrieved on 2016-04-08]
- "Formulation for DMEM without Phenol Red ATCC 30-2601 ATCC (American Type Culture Collection)", , 8 April 2016 (2016-04-08), XP055263764, Retrieved from the Internet: URL:http://www.atcc.org/~/media/0DB843DD93 B14D45A1C2C3554D3527A7.ashx [retrieved on 2016-04-08]
- Anonymous: "Phosphate-buffered saline - Wikipedia, the free encyclopedia", , 18 February 2016 (2016-02-18), XP055263772, Retrieved from the Internet: URL:https://en.wikipedia.org/wiki/Phosphat e-buffered_saline#cite_note-1 [retrieved on 2016-04-08]

## Description

### FIELD OF INVENTION

The present invention relates to a cell culture medium, especially for culturing human autologous fibroblasts intended for skin transplantation into the field of aesthetics or regenerative medicine.

### BACKGROUND ART

In aesthetic and regenerative medicine, the use of autologous fibroblasts transplantation, in jargon also called "Autotransplantation", is a technique employed for quite some time.

With aging, the number of active fibroblasts in the skin tends to decrease, as well as the metabolic capabilities thereof. The increase of cells due to transplantation of autologous fibroblasts with the ability to synthesize the extracellular matrix allows for counteracting the effects of skin aging.

Indeed, it was clinically demonstrated that, in patients who were injected with cultures of autologous fibroblasts from explants of the same patients and cultivated in vitro, they have improved the biomechanical properties of skin of above-mentioned patients, as demonstrated with different kinds of clinical trials, such as optical profilometry, vacuum cutometry, and the VISIA^{®} photometric analysis.

Indeed, the fibroblasts injected were able to synthesize the extracellular matrix for at least 12 months.

In areas of the skin of the patients who have undergone treatment actually, it is noted an increase of collagen and a general thickening of dermis (up to 60% in 12 months), an increase of elasticity of the skin (25% in the periorbital area) and a general reduction of wrinkles equal to 50% (V.Zorin et al "Clinical-instrumental and morphological evaluation of the effect of autologous dermal fibroblasts administration" Journal of Tissue Engineering and Regenerative Medicine Article first published online: 19 DEC 2014b DOI: 10.1002/term.1976).

Although the good results obtained with this technique, it finds it hard to take hold for a number of criticalities, first of all the cell culture medium.

In fact, it is well known that the culture media in which cells such as fibroblasts grows are not always suitable to be injected into humans.

In fact, it is known that the cells exhibit an excellent cell viability and proliferation in the presence of serum and the serum generally employed is the fetal bovine serum (PBS). This type of substances can be immunogen and therefore cause allergic reactions in some subjects. Other types of cell culture media such as DMEM have extremely high amounts of glucose that can be dangerous in subjects with overt diabetes or with diabetic tendencies.

It's felt the need to have cell culture media which do not present the aforesaid drawbacks, i.e. that are serum-free while containing a lower content of glucose, compared to the cell culture media such as DMEM.

On the other hand, it is known a physiological saline solution, called Tyrode's solution, which allows for storing organs for transplantation at low temperatures for 10-12 hours.

This aqueous solution consists of the following components in the respective concentrations

**Standard Tyrode's solution**

| | |
|---|---|
| NaCI | 137mM |
| KCl | 2.7mM |
| D-Glucose | 5.5mM |
| NaHCO₃ | 12mM |
| NaH₂PO₄ | 0.2mM |
| MgCl₂ | 1mM |
| CaCl₂ | 1.8mM |

Alexis Carrell et al in "Action on fibroblasts of extracts of homologous and heterologous tissues" (Anat. Rec. J. Exp. Med. Exp. Med..Med. Exp. Med.Y Exp, Med. Exp. Med. Exp. Med Exp. Med.1, Janaury 1921 pages 759-1916 XP055263349 Retrieved from the Internet: URL: http://jem.rupress .org/content /38/5/499.full.pdf#page=1eview=FitH) disclose the culture of fibroblast in Tyrode on its own or with embryonic extracts added.

S. R. Smith et al in "A multicenter,double blind placebo controlled trial of autologous fibroblast therapy for the treatment of nasolabial fold wrinkles"(Dermatologic surgery, Wiley-Blackwell Publishing Inc. New York US, vol.38, NO.7, 1 July 2012 (2012-07-01) XP002714258, ISSN 1076-0512, retrieved from the Internet: URL: http://onlinelibrary.wiley .com / doi/ 19.1111/j. 1524-4725.2012.02349.x/pdf [retrieved on 2012-03-121) disclose the preparation of human autologous fibroblasts for the treatment of wrinkles. The fibroblast are cultured in IMDM comprising fetal bovine serum, rinsed in PBS or PBS and DMEM. V. Zorin et al disclose the preparation of human autologous fibroblasts for the treatment of ageing skin. The fibroblasts were cultured in DMEM comprising 10% FBS and washed for 24h to remove any traces of growth media supplements (V.Zorin et al "Clinical -instrumental and morphological evaluation of the effect of autologous dermal fibrblasts administration "Journal of Tissue of Engineering and regenerative Medicine, 19 December 2014XP055263248 ISSN: 1932-6254, DOI: 10.1002/term 1976.

The composition of IMDM and DMEM are reported respectively in : "Formulation for Iscove's Modified Dulbecco's medium (IMDM) ATTC, April 8, 2016 (2016-04-08) XP055263745, retrieved from the Internet: URL: http//www.lgcstandards-atcc.org/≈/media/Attachments/E/9/7/E/4893.ashx [retrieved on 2016-04-08] and in "Formulation for DMEM without phenol Red ATCC30-2601 ATTC (American type cultrure collection)" , 8 April 2016 (2016-04-08) XP055263764, retrieved from the Internet http//www-attc.ore/≈/media/0DB843DD93B14D45EA1C2C3554D3527A77.ashx [retrieved on 2016-04-081

### SUMMARY OF THE INVENTION

The applicant has now found a saline solution, which, when employed as cell culture medium, in particular for culturing fibroblasts, maintains up to 30% of viable cells for a 24 hours' incubation period in said liquid. Also this type of saline solution allows for a markedly higher cell growth than a culture medium containing only PBS.

Therefore, an object of the present invention is an aqueous saline solution as claimed in claim 1, containing
a) a mixture of mineral salts consisting of sodium chloride, potassium chloride, sodium bicarbonate, sodium dihydrogen phosphate, magnesium chloride and calcium chloride,
b) D-glucose and
c) at least a water-soluble vitamin,
   wherein: the concentration of b) D-glucose is < 6mM and said at least one water soluble is a combination of vitamin C and B6

A further object of the present invention is a serum-free cell culture medium consisting of the above-mentioned physiological solution. autologous fibroblasts expanded in the culture medium according to the present invention can be advantageously used in skin transplantation in aesthetic or regenerative medicine.

### DESCRIPTION OF FIGURES

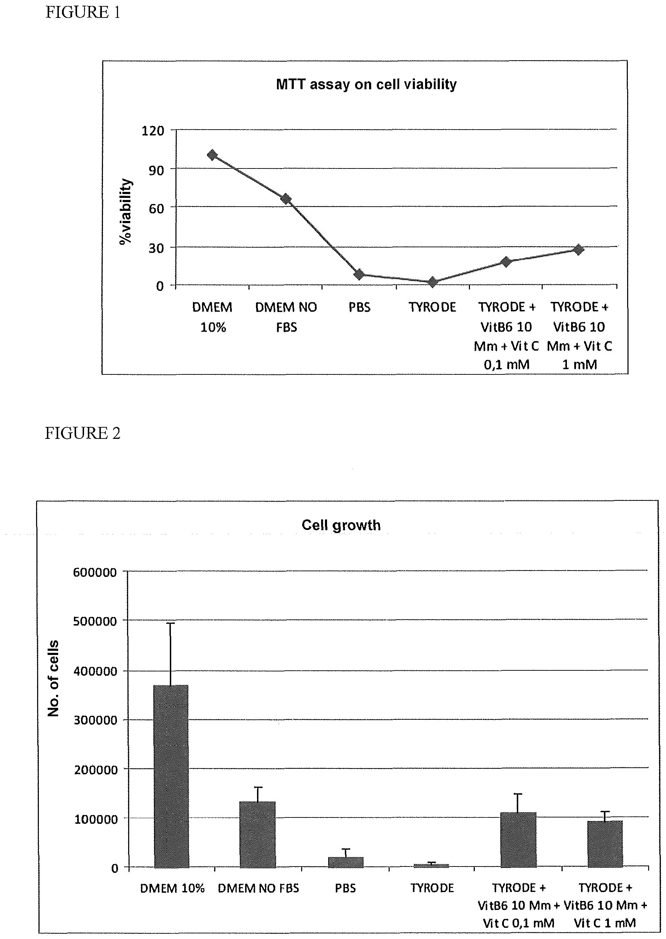

Figure 1 shows in graphical presentation the MTT assay results on % cell viability of fibroblasts after 24 hours of incubation in DMEM, DMEM + PBS, PBS only, conventional Tyrode's solution, a solution according to the present invention in the presence of Vitamin C (0.1mM) and Vitamin B6 (10mM) and a solution according to the present invention in the presence of Vitamin C (ImM), and Vitamin B6 (10mM), respectively.
Figure 2 shows proliferation of human fibroblasts (cell counting) after a 24 hours' incubation in the following culture media: DMEM only, DMEM + PBS, PBS only, conventional Tyrode's solution, a solution according to the present invention in the presence of Vitamin C (0.1mM), Vitamin B6 (10mM) and a solution according to the present invention in the presence of Vitamin C (ImM), Vitamin B6 (10mM).

### DETAILED DESCRIPTION OF THE INVENTION

Preferably the saline solution of the invention has a concentration of the component b), i.e. D-glucose, of 5,5mM.

Preferably for the objects of the present invention by Vitamin B6 it is intended pyridoxine, pyridoxamine hydrochloride, pyridoxal o pyridoxal phosphate. Preferably, the Vitamin C is present at a concentration between 0.05mM and 2mM and the Vitamin B6 is present at a concentration ranging between 5mM and 20mM.

According to a particularly preferred embodiment of the invention, the saline solution of the present invention consists of the following components in their respective millimolar concentrations:

| | | |
|---|---|---|
| NaCl | 130 | mM, |
| KCl | 2.68 | mM, |
| D-Glucose | 5.5 | mM, |
| NaHCO₃ | 11.9 | mM, |
| NaH₂PO₄ | 0.46 | mM, |
| MgCl₂ | 1.05 | mM, |
| CaCl₂ | 1.8 | mM, |
| Vitamin C | 0.1 - 1 | mM, |
| Vitamin B6 | 10 | mM. |

Experimental evidences of cell viability and cellular proliferation of fibroblasts carried on cell cultures in DMEM, DMEM + PBS, only PBS, conventional Tyrode's solution, a solution according to the present invention in the presence of Vitamin C (0.1mM), Vitamin B6 (10mM) and a solution according to the present invention in the presence of Vitamin C (ImM), and Vitamin B6 (10mM), respectively, are reported for illustrative purposes herein below.

### EXAMPLE

### Method

The cells (primary human dermal fibroblasts) were grown in standard amplification conditions (37° C, 5% CO₂, 95% humidity in the incubator) in the following culture liquids
- DMEM
- DMEM supplemented with antibiotics and 10% serum, in the culture liquid containing PBS serum (10%)
- Standard Tyrode
- Two Tyrode's solutions modified according to the invention and containing each Vitamin B6 at a concentration of 10mM and Vitamin C at a concentration of 0.1 and 1mM, respectively.

Conventional or standard Tyrode's formulations and modified Tyrode's formulations according to the present invention and examined in the present example are shown in the following table herein below.

| Component | Tyrode's solution modified according to the present invention | Standard Tyrode |
|---|---|---|
| NaCI | 130mM | 137mM |
| KCl | 2.68mM | 2.7mM |
| D-Glucose | 5.5mM | 5.5mM |
| NaHCO3 | 11.9mM | 12mM |
| NaH₂PO₄ | 0.46mM | 0.2mM |
| MgCl2 | 1.05mM | 1mM |
| CaCl2 | 1.8mM | 1.8mM |
| Vit C | 0.1mM or 1mM | Absent |
| Vit B6 | 10mM | Absent |

At the time of the experiment, the cells, detached by trypsin treatment and after centrifugation and washing in sterile buffer (PBS), were suspended in the various solutions prepared and then counted with an automatic cell counter.

During the 24 h incubation, the cells were maintained in the incubator as for the amplification step.

The cell cultures were then assessed for viability (MTT assay) and proliferation (cell counting).

The results of the above tests are reported in Figures 1 and 2, respectively.

As can be seen after a 24 hours' incubation in serum-free media (PBS and Tyrode's standard) the cells have in fact completely lost their viability. In the case of culture medium DMEM with serum, the cells have more than 90% of survival that drops to just over 60% in DMEM without serum, however in the presence of high doses of amino acids, vitamins, and 25mM glucose, which is five times the standard concentration. The modified Tyrode's solutions according to the present invention instead maintain the cells viable up to 30% after 24 hours, a much better result than the standard Tyrode's solution, but also than the culture liquid containing PBS only.

The two modified Tyrode's solution, object of the present invention and examined, show a significantly better proliferation compared to standard formulation containing PBS only and the standard Tyrode, although obviously lower than DMEM formulations that are markedly much more rich in amino acids and micronutrients.

## Claims

1. A saline aqueous solution comprising:
a) a mixture of biologically acceptable mineral salts consisting of sodium chloride, potassium chloride, sodium bicarbonate, sodium dihydrogen phosphate, magnesium chloride and calcium chloride,
b) D-glucose and
c) at least a water-soluble vitamin,
wherein:
i) the concentration of b) glucose is < 6mM,
ii) said at least one water soluble vitamin c) is an association of vitamin C and B6.

2. The saline physiological solution according to claim 1, wherein the concentration of b) glucose is 5.5mM.

3. The saline aqueous solution according to claim 1 or 2, wherein said Vitamin C is present at a concentration between 0.05mM and 2mM and said Vitamin B6 is present at a concentration between 5mM and 20mM.

4. The saline aqueous solution according to any one of claimsl-3, **characterized in that** said solution is a modified Tyrode's solution and consists of the following components in the respective millimolar concentrations:
| | | |
|---|---|---|
| NaCl | 130 | mM, |
| KCl | 2.68 | mM, |
| D-Glucose | 5.5 | mM, |
| NaHCO₃ | 11.9 | mM, |
| NaH₂PO₄ | 0.46 | mM, |
| MgCl₂ | 1.05 | mM, |
| CaCl₂ | 1.8 | mM, |
| Vitamin C | 0.1 - 1 | mM, |
| Vitamin B6 | 10 | mM. |

5. A serum-free cell culture medium consisting of the saline aqueous solution according to any one of claims 1-4.

## Patentansprüche

1. Wässrige Kochsalzlösung, umfassend:
a) eine Mischung aus biologisch annehmbaren Mineralsalzen, bestehend aus Natriumchlorid, Kaliumchlorid, Natriumbicarbonat, Natriumdihydrogenphosphat, Magnesiumchlorid und Calciumchlorid,
b) D-Glucose und
c) mindestens ein wasserlösliches Vitamin,
wobei:
i) die Konzentration von b) Glukose < 6 mM ist,
ii) dass mindestens ein wasserlösliches Vitamin c) eine Assoziation von Vitamin C und B6 ist.

2. Physiologische Kochsalzlösung nach Anspruch 1, wobei die Konzentration von b) Glucose 5,5 mM ist.

3. Die wässrige Kochsalzlösung nach Anspruch 1 oder 2, in der das Vitamin C mit einer Konzentration zwischen 0,05mM und 2mM vorhanden ist und das Vitamin B6 mit einer Konzentration zwischen 5mM und 20mM vorhanden ist.

4. Die wässrige Kochsalzlösung nach einem der Ansprüche 1 bis 3, **dadurch gekennzeichnet, dass** es sich bei der Lösung um eine modifizierte Tyrode-Lösung handelt und sie aus den folgenden Bestandteilen in den jeweiligen millimolaren Konzentrationen besteht:
| | | |
|---|---|---|
| NaCl | 130 | mM, |
| KCl | 2,68 | mM, |
| D-Glucose | 5,5 | mM, |
| NaHCO₃ | 11,9 | mM, |
| NaH₂PO₄ | 0,46 | mM, |
| MgCl₂ | 1,05 | mM, |
| CaCl₂ | 1,8 | mM, |
| Vitamin C | 0,1 - 1 | mM, |
| Vitamin B6 | 10 | mM. |

5. Serumfreies Zellkulturmedium, das aus der wässrigen Kochsalzlösung nach einem der Ansprüche 1 bis 4 besteht.

## Revendications

1. Solution aqueuse saline comprenant :
a) un mélange de sels minéraux biologiquement acceptables constitué de chlorure de sodium, de chlorure de potassium, de bicarbonate de sodium, de dihydrogénophosphate de sodium, de chlorure de magnésium et de chlorure de calcium,
b) D-glucose et
c) au moins une vitamine hydrosoluble,
dans laquelle :
i) la concentration de b) glucose est < 6mM,
ii) ladite au moins une vitamine hydrosoluble c) est une association de vitamine C et B6.

2. Solution physiologique saline selon la revendication 1, dans laquelle la concentration de b) glucose est de 5,5 mM.

3. Solution aqueuse saline selon la revendication 1 ou 2, dans laquelle ladite vitamine C est présente à une concentration comprise entre 0,05 mM et 2 mM et ladite vitamine B6 est présente à une concentration comprise entre 5 mM et 20 mM.

4. Solution aqueuse saline selon l'une quelconque des revendications 1-3, **caractérisée en ce que** ladite solution est une solution de Tyrode modifiée et est composée des composants suivants dans les concentrations millimolaires respectives :
| | | |
|---|---|---|
| NaCl | 130 | mM, |
| KCl | 2,68 | mM, |
| D-Glucose | 5,5 | mM, |
| NaHCO₃ | 11,9 | mM, |
| NaH₂PO₄ | 0,46 | mM, |
| MgCl₂ | 1,05 | mM, |
| CaCl₂ | 1,8 | mM, |
| Vitamine | C 0,1 - 1 | mM, |
| Vitamine B6 | 10 | mM. |

5. Milieu de culture de cellules exempt de sérum, constitué de la solution aqueuse saline selon l'une quelconque des revendications 1 à 4.
